# EUROPEAN PATENT APPLICATION

(11) **EP 1 652 853 A1**
(43) Date of publication of application: **03.05.2006**
(21) Application number: 04748012.4
(22) Date of filing: 21.07.2004
(51) Int. Cl.: C07H 19/23, A61K 31/7056, A61P 35/00

(54) **CRYSTALLINE 6-N-PYRIDYLMETHYLAMINOINDOLOCARBAZOLE COMPOUNDS**

(30) Priority: 24.07.2003 WO PCT/JP03/09393
(71) Applicant: BANYU PHARMACEUTICAL CO., LTD., Chuo-ku, Tokyo 103-8416 (JP)
(72) Inventor: IMAMURA, Hideaki, c/o BANYU PHARMACEUTICAL CO. LTD, Tsukuba-shi, Ibaraki, 3002611 (JP); SUNAMI, Satoshi, c/o BANYU PHARMACEUTICAL CO., LTD, Tsukuba-shi, Ibaraki, 3002611 (JP); HIRANO, Atsushi, c/o BANYU PHARMACEUTICAL CO., LTD, Tsukuba-shi, Ibaraki, 3002611 (JP); OHKUBO, Mitsuru, c/o BANYU PHARMACEUTICAL CO., LTD, Tsukuba-s hi, Ibaraki, 3002611 (JP); AKAO, Atsushi, c/o BANYU PHARMACEUTICAL CO., LTD., Okazaki-shi, Aichi, 4440858 (JP)
(74) Representative: Rollins, Anthony John
(86) International application number: PCT/JP2004/010741
(87) International publication number: WO 2005/010019

(57) **Abstract**

The present invention relates to crystalline free base compound having the following formula:

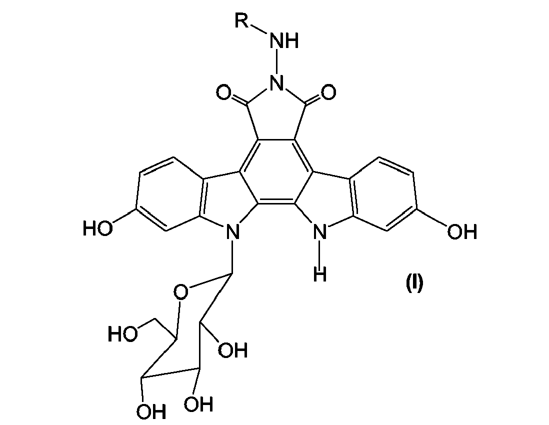

wherein R is an unsubstituted pyridyl methyl group or a pyridyl methyl group substituted by a hydroxy methyl group; or a pharmaceutically acceptable salt or a solvate thereof.

## Description

### Technical Field

The present invention relates to a free base of an indolopyrrolocarbazole derivative in a novel crystalline form or a pharmaceutically acceptable salt or a solvate thereof, which is useful in the field of pharmaceuticals and, to be more specific, inhibits the growth of tumor cells to exhibit an anti-tumor effect and also to a process for producing the same, a pharmaceutical composition containing the same as an active ingredient.

### Background Art

We have found novel indolopyrrolocarbazole derivatives having an anti-cancer activity and filed patent applications for such a series of the compounds (U. S. Patent No. 5591842, U. S. Patent No. 5668271, U. S. Patent No. 5804564, U. S. Patent No. 5922860, International Gazette No. 95/30682, International Gazzette No. 96/04293, International Gazzette No. 98/0743, European Unexamined Patent Publication No. 0528030, JP-A-10-245390, etc.).

Among the above, described in the specification of JP-A-10-245390 is a compound represented by the following formula: 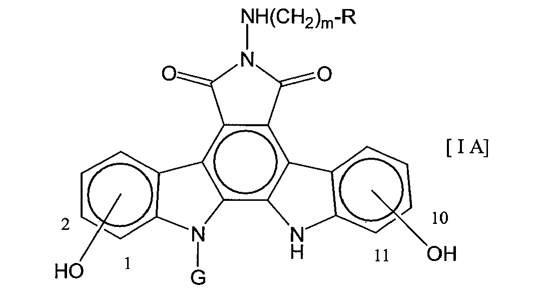 wherein, R is a phenyl group, naphthyl group, pyridyl group, furyl group or thienyl group having one or two substituent(s) selected from the group consisting of hydroxy, lower alkoxy, hydroxy-lower alkyl and hydroxy-lower alkenyl, with the proviso that, when R has a lower alkoxy group as a substituent, R also has another substituent selected from the group consisting of hydroxy, lower alkoxy, hydroxy-lower alkyl and hydroxy-lower alkenyl; m is an integer of 1 to 3; G is a β-D-glucopyranosyl group; and the substituted positions of hydroxy group on the indolopyrrolocarbazole ring are 1- and 11-positions or 2-and 10-positions. However, in that specification, there is neither description nor suggestion for the crystalline form of the said compounds. In fact, it has been confirmed that, when the compounds of the Examples are synthesized and isolated by the process mentioned in the said specification, they are amorphous.

When the above compounds are actually marketed as anti-tumor agents, it is desired to be in a crystalline form in view of the physico-chemical stability of the compounds. Particularly, the amorphous solids of the above compounds are insufficient in stability and, when they are preserved for a long period under ordinary conditions, they are discolored resulting in deterioration of purity. Even if the above compounds are put into the market as amorphous solid or liquid preparations, complicated purifying steps are necessary for manufacturing the amorphous solid as substantially pure one and there are various problems in actual putting into the market without purifying steps by means of crystallization.

As such, although it is quite important to prepare the above indolocarbazole compounds in a crystalline form, there have been no detailed studies for crystals of the above compounds.

### Disclosure of the Invention

The present inventors have conducted intensive studies for solving the above problems due to amorphousness and, as a result, they have found the compounds in a crystalline form among the compounds represented by the above formula (IA) to complete the present invention.

The present invention relates to a free base of a crystalline 6-N-pyridylmethylamino-12,13-dihydro-2,10-dihydroxy-12-β-D-glucopyranosyl-5H-indolo[2,3-a]pyrrolo [3,4-c]carbazole- 5,7(6H)-dione represented by the following formula (I): 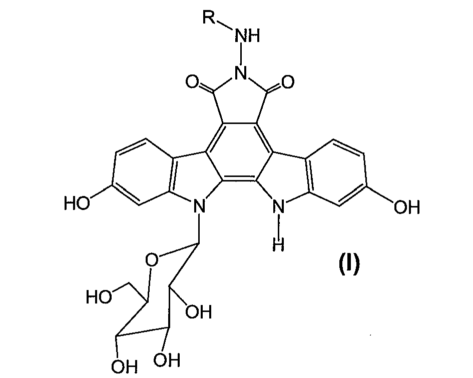 wherein R is a pyridylmethyl group which is not substituted or is substituted with a hydroxymethyl group, a pharmaceutically acceptable salt thereof or a solvate thereof.

Preferably, the present invention relates to a free base of a crystalline compound represented by the following formula (II): 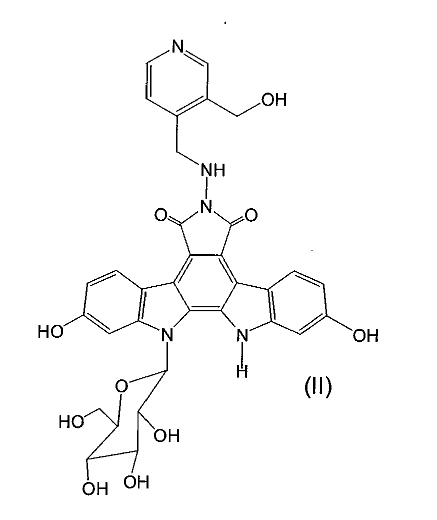 a pharmaceutically acceptable salt thereof or a solvate thereof; a free base of a crystalline compound represented by the following formula (III): 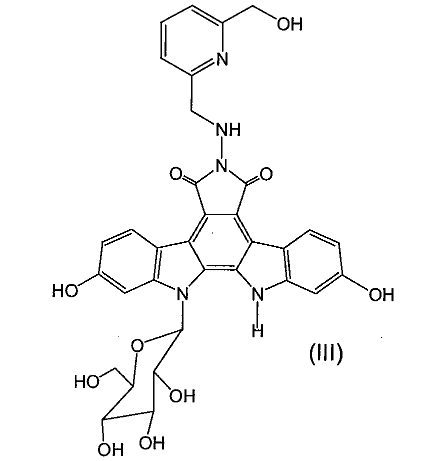 a pharmaceutically acceptable salt thereof or a solvate thereof; or a free base of a crystalline compound represented by the following formula (IV): 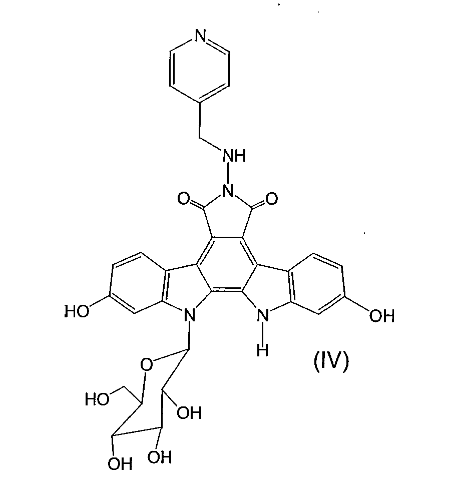 a pharmaceutically acceptable salt thereof or a solvate thereof.

Comparing the free base of the compound represented by the above formula (I) in a crystalline form with the pharmaceutically acceptable salt (hereinafter, referred to as "intramolecular salt") of the compound represented by the formula (I) in a crystalline form, the intramolecular salt of the compound represented by the formula (I) in a crystalline form is more desirable. This is because, particularly with regard to injection preparation, there is expected to be no need to add large amount of additives in terms of improved solubility and preparation of formulations.

In JP-A-10-245390, there is no disclosure for any specific structure and manufacturing process for the compound represented by the formula (IV) and, therefore, it is recognized to be a novel compound. The manufacturing process of the compound is mentioned in Example 7-1.

The present invention more preferably relates to: hydrochloride, sulfate salt or methanesulfonate salt of the compound represented by the formula (II) in a crystalline form; or hydrochloride, sulfate salt or methanesulfonate salt of the compound represented by the formula (III) in a crystalline form; or hydrochloride, sulfate salt or methanesulfonate salt of the compound represented by the formula (IV) in a crystalline form.

Also, the present invention preferably relates to a solvate of the compound represented by the formula (II), (III) or (IV) in a crystalline form.

The present invention more preferably relates to an ethanol solvate of methanesulfonate salt of the compound represented by the formula (II) in a crystalline form.

The present invention relates to a pharmaceutical composition containing a free base of the compound represented by the above formula (I) in a crystalline form, a pharmaceutically acceptable salt thereof or a solvate thereof as an active ingredient together with a pharmaceutically acceptable carrier or diluent.

The present invention also relates to an anti-tumor agent containing a free base of the compound represented by the above formula (I) in a crystalline form, a pharmaceutically acceptable salt thereof or a solvate thereof as an active ingredient together with a pharmaceutically acceptable carrier or diluent.

The present invention further relates to an anti-tumor agent for injection using the compound of the above formula (I) in a crystalline form together with a pharmaceutically acceptable carrier or diluent.

Still further, the present invention preferably relates to an anti-tumor agent for injection using the compound represented by the above formulae (II), (III) or (IV) in a crystalline form together with a pharmaceutically acceptable carrier or diluent.

The term "crystalline form" as used herein means a solid state of which internal structure is made of a three-dimensionally regular repetition of the constituting elements, including crystal polymorphs. On the other hand, the term "amorphous" means an amorphous state which is not a crystalline state.

The term "free base" as used in the present specification means a state where a base molecule is not bonded to an acid or hydrogen ion.

The term "pharmaceutically acceptable salt" used herein include, for example, an addition salt with inorganic acids such as hydrochloric acid, sulfuric acid, hydrobromic acid, nitric acid, phosphoric acid and perchloric acid; organic acids such as acetic acid, maleic acid, fumaric acid, tartaric acid, citric acid, lactic acid, succinic acid, ascorbic acid and trifluoroacetic acid; sulfonic acids such as methanesulfonic acid, benzenesulfonic acid and p-toluenesulfonic acid. Preferably, it includes an addition salt with hydrochloric acid, sulfuric acid, phosphoric acid, acetic acid, fumaric acid, tartaric acid, citric acid, lactic acid, succinic acid, methanesulfonic acid or benzenesulfonic acid; and more preferably it includes an addition salt with hydrochloric acid, sulfuric acid or methanesulfonic acid.

The term "solvate" as used herein means a compound to which a solvent is bonded and includes, for example, ethanol solvate and hydrate.

The term "pharmaceutically acceptable carrier or diluent" as used herein includes solvents such as water, physiological saline solutions, alcohols (e.g., ethanol), glycerols and vegetable oil; additives such as excipients, bases, disintegrating agents, binders, lubricants, moisturizers, stabilizers, emulsifiers, dispersing agents, preservatives, sweeteners, coloring agents, corrigents, aromatizing agents, buffers, solubilizers, antiseptic agents, salts for changing osmotic pressures, coating agents or antioxidants.

The term "anti-tumor agent" ("anti-cancer agent") as used herein means a "preparation" for the treatment of patients suffering from "cancer" ("tumor") and the term "cancer" is solid cancer and cancer of blood-forming organ. The term "solid cancer" includes, for example, brain cancer, cephalic/cervical cancer, esophageal cancer, thyroid cancer, small-cell lung cancer, non-small-cell lung cancer, breast cancer, stomach cancer, cancer of gallbladder and bile duct, hepatic cancer, pancreatic cancer, colon cancer, rectal cancer, ovarian cancer, cholioepithelium cancer, glomerular cancer, cervical cancer, cancer of renal pelvis and urinary duct, bladder cancer, prostate cancer, penile cancer, testicular cancer, fetal cancer, viral tumor, skin cancer, malignant melanoma, neuroblastoma, osteosarcoma, Ewing's sarcoma and soft tissue sarcoma; preferably colon cancer, small-cell lung cancer, non-small-cell lung cancer, bladder cancer, cephalic/cervical cancer, stomach cancer, pancreatic cancer, hepatic cancer and ovarian cancer; and more preferably, colon cancer, non-small-cell lung cancer and cephalic/cervical cancer. On the other hand, The term "cancer of blood-forming organ" includes, for example, acute leukemia, chronic lymphocytic leukemia, chronic myelogenous leukemia, erythrosis, malignant lymphoma, multiple myeloma and non-Hodgkin's lymphoma.

The term "Preparation" includes oral and parenteral preparations. The term "oral preparation" includes, for example, tablets, capsules, powder and granules, while the term "parenteral preparation" includes, for example, sterilized liquid preparations such as solution and suspensions, and to be more specific, injections and drips; preferably intravenous injections and intravenous drips; and, more preferably intravenous drips. With regard to those intravenous injections and drips, any form where powder *per se* or its mixture with an appropriate carrier (additive) is dissolved just before use may be applied in addition to those previously dissolved.

Next, a process for the manufacture of a compound in a crystalline form represented by the formula (I) will be mentioned.

The present invention relates to a process for the manufacture of a compound represented by the above formula (I) in a crystalline form, comprising:
- the step of adding, to an compound represented by the above formula (I) in an amorphous form, an organic solvent solution which contains 1- to 100-equivalent(s) of the corresponding acid or which may contain an appropriate amount of acetic acid so that its concentration becomes 50 mg/L to 1 g/L;
- the step of subjecting the resulting solution to be heated to reflux;
- if necessary, the step of filtering the resulting solution to remove insoluble matter;
- the step of evaporating the solvent from the resulting solution to concentrate;
- the step of subjecting a suspension of the resulting solid to be heated to reflux;
- the step of cooling the resulting suspension down to 0°C to 35°C; and
- the step of isolating the resulting crystals.

More specific embodiments of the process are the following Manufacturing Methods 1 and 2.

### Manufacturing Method 1 (In the case of crystals of the intramolecular salt of the compound represented by the above formula (I))

To the compound represented by the formula (I) in an amorphous form, added is a solution selected from a group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solution thereof, containing from 1- to 100-equivalent(s), preferably from 5- to 20-equivalents, of the corresponding acid (hydrochloric acid, sulfuric acid, methanesulfonic acid, etc.); preferably a solution selected from the group consisting of methanol, ethanol, 1-propanol, 2-propanol, 1-butanol, 2-butanol and a mixed solvent thereof; or, more preferably, a methanolic solution so as to adjust the concentration of the solution to 50 mg/L to 1 g/L followed by heating to reflux for 5 minutes to 200 hours, preferably for 10 to 20 hours or, more preferably for 15 hours.

When there is anything which is not completely dissolved in the resulting solution, the insoluble matter is removed by filtration under ordinary pressure or *in vacuo*. After that, the solvent is concentrated by evaporating under ordinary pressure or *in vacuo* so that the amount of the solvent is reduced to 1/3 to 1/10 of the initial amount thereof, resulting in a solid being separated out.

A suspension *per se* of the separated solid is heated to reflux for 1 to 200 hour(s), preferably 10 to 20 hours or, more preferably, 15 hours.

The resulting suspension is cooled down to 0°C to 35°C and the resulting solid is filtered under ordinary pressure or *in vacuo* and washed for one to several times with an appropriate amount of a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; preferably a solvent selected from the group consisting of methanol, ethanol, 1-propanol, 2-propanol, 1-butanol, 2-butanol and a mixed solvent thereof; or, more preferably methanol or ethanol.

The resulting solid is dried *in vacuo* at 25°C to 80°C, preferably at 40°C, for 0.5 to 100 hour(s) to give a solid. The resulting solid is suspended in a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dihloroethane and a mixed solvent thereof; preferably a solvent selected from the group consisting of methanol, ethanol, 1-propanol, 2-propanol, 1-butanol, 2-butanol and a mixed solvent thereof; or, more preferably, ethanol, and then heated with stirring at 40°C to 130°C, preferably at 70°C to 80°C, or, more preferably at 75°C, for 0.1 to 200 hour(s), preferably for 15 to 25 hours or, more preferably for 20 hours.

The resulting reaction solution is cooled down to 0°C to 35°C or, preferably to room temperature, and then the resulting solid is filtered under ordinary pressure or *in vacuo*, and washed for one to several times with an appropriate amount of a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; preferably a solvent selected from the group consisting of methanol, ethanol, 1-propanol, 2-propanol, 1-butanol, 2-butanol and a mixed solvent thereof; or, more preferably ethanol or 2-propanol.

The resulting solid is dried *in vacuo* at 25°C to 80°C, preferably at 40°C to 60°C or, more preferably at 50°C, for 0.5 to 100 hour(s) to give the desired intramolecular salt crystals of the compound represented by the formula (I).

### Manufacturing Method 2 (In the case of crystals of the free base of the compound represented by the above formula (I))

To the compound represented by the formula (I) in an amorphous form, added is a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solution thereof; preferably a solvent selected from the group consisting of methanol, ethanol, 1-propanol, 2-propanol, 1-butanol, 2-butanol and a mixed solvent thereof; or, more preferably, ethanol, or alternatively a solution of the above solvent containing 0.1- to 100-equivalent(s) of acetic acid so as to adjust the concentration of the solution to 50 mg/L to 1 g/L, followed by heating to reflux for 5 minutes to 200 hours, preferably for 0.25 to 15 hours. When there is anything which is not completely dissolved in the resulting solution, the insoluble matter is removed by filtration under ordinary pressure or *in vacuo*.

Next, the solvent is concentrated by evaporating under ordinary pressure *or in vacuo* so that the amount of the solvent is reduced to 1/3 to 1/10 of the initial amount thereof, resulting in a solid being separated out. A suspension *per se* of the separated solid is heated to reflux for 0 minute to 200 hour(s) or, preferably 2.5 to 15 hours.

The resulting suspension is cooled down to 0°C to 35°C or, preferably to room temperature, and then the resulting solid is filtered under ordinary pressure *or in vacuo* and washed for one to several times with an appropriate amount of solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; preferably a solvent selected from a group consisting of methanol, ethanol, 1-propanol, 2-propanol, 1-butanol, 2-butanol and a mixed solvent thereof; or, more preferably methanol, ethanol or 2-propanol.

The resulting solid is dried *in vacuo* at 25°C to 80°C, preferably at 40°C to 60°C, more preferably 50°C, for 0.5 to 100 hour(s) to give a solid. The resulting solid is suspended in a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; or, preferably a solvent selected from a group consisting of methanol, ethanol, 1-propanol, 2-propanol, 1-butanol, 2-butanol and a mixed solvent thereof, and then heated with stirring at 40°C to 130°C or, preferably at 70°C to 80°C, for 0.1 to 200 hour(s), preferably for 15 to 25 hours or, more preferably, for 20 hours.

The resulting reaction solution is cooled down to 0°C to 35°C or, preferably at 35°C, and then the resulting solid is washed for one to several times with an appropriate amount of solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane and 1,2-dichloroethane and a mixed solvent thereof; preferably a solvent selected from a group consisting of methanol, ethanol, 1-propanol, 2-propanol, 1-butanol, 2-butanol and a mixed solvent thereof; or, more preferably methanol or, ethanol or 2-propanol. The resulting solid is dried *in vacuo* at 25°C to 80°C, preferably at 40°C to 60°C or, more preferably at 50°C, for 0.5 to 100 hour(s) to give the desired crystals of free base of the compound represented by the formula (I).

The present invention further relates to a process for the manufacture of a compound represented by the formula (I) in a crystalline form, comprising:
- the step of suspending the compound represented by the formula (I) in an amorphous form in an organic solvent solution which contains 1- to 100-equivalent(s) of the corresponding acid or which may contain an appropriate amount of acetic acid;
- the step of heating the resulting solution from 40°C to 130°C with stirring;
- the step of cooling the resulting solution down to 0°C to 35°C; and
- the step of isolating the resulting crystals.

More specific embodiments of the said process are the following Manufacturing Methods 3 and 4.

### Manufacturing Method 3 (In the case of crystals of the intramolecular salt of the compound represented by the above formula (I))

The compound represented by the formula (I) in an amorphous form is suspended in a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solution thereof containing from 1- to 100-equivalent(s) or, preferably 2-equivalents, of the corresponding acid (hydrochloric acid, sulfuric acid, methanesulfonic acid, etc.); preferably a solvent selected from the group consisting of methanol, ethanol, 1-propanol, 2-propanol, 1-butanol, 2-butanol and a mixed solvent thereof; or, more preferably ethanol, followed by heating with stirring at 40°C to 130°C or, preferably at 75°C to 80°C, for 0.1 to 200 hour(s) or, preferably for 15 to 168 hours.

The resulting suspension is cooled down to 0°C to 35°C, and then the resulting solid is filtered under ordinary pressure *or in vacuo* and washed for one to several times with an appropriate amount of a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; preferably a solvent selected from a group consisting of methanol, ethanol, 1-propanol, 2-propanol, 1-butanol, 2-butanol and a mixed solvent thereof; or, more preferably ethanol or 2-propanol.

The resulting solid is dried *in vacuo* at 25°C to 80°C, preferably at 40°C to 60°C or, more preferably at 50°C, for 0.5 to 100 hour(s) to give the desired intramolecular salt of the compound represented by the formula (I).

### Manufacturing Method 4 (In the case of crystals of the free base of the compound represented by the above formula (I))

The compound represented by the formula (I) in an amorphous form is suspended in an appropriate amount of a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solution thereof; preferably a solvent selected from a group consisting of methanol, ethanol, 1-propanol, 2-propanol, 1-butanol, 2-butanol and a mixed solvent thereof; or alternatively the above solvent containing 0.1- to 100-equivalent(s) of acetic acid followed by heating with stirring at 40°C to 130°C, preferably at 75°C to 80°C, for 0.1 to 200 hour(s), preferably for 15 to 24 hours.

The resulting suspension is cooled down to 0°C to 35°C or, preferably to room temperature, and then the resulting solid is filtered under ordinary pressure or *in vacuo* and washed for one to several times with an appropriate amount of a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; preferably a solvent selected from the group consisting of methanol, ethanol, 1-propanol, 2-propanol, 1-butanol, 2-butanol and a mixed solvent thereof; or, more preferably ethanol or 2-propanol.

The resulting solid is dried *in vacuo* at 25°C to 80°C, preferably at 40°C to 60°C or, more preferably 50°C, for 0.5 to 100 hour(s) to give the desired crystals of the free base of the compound represented by the formula (I).

The present invention still further relates to a process for the manufacture of a compound represented by the formula (I) in a crystalline form, comprising:
- the step of adding the compound represented by the formula (I) in amorphous form to water or to an aqueous solution containing 1- to 100-equivalent(s) of the corresponding acid;
- the step of heating the resulting solution at 40°C to 130°C with stirring;
- if necessary, the step of filtering the resulting solution to remove the insoluble matter;
- the step of cooling the temperature of the resulting reaction solution down to 0°C to 35°C with stirring; and
- the step of isolating the resulting crystals.

A more specific embodiment of the process is the following Manufacturing Method 5.

### Manufacturing Method 5 (In the case of crystals of the free base and crystals of the intramolecular salt of the compound represented by the above formula (I))

To the compound represented by the formula (I) in an amorphous form, added is an appropriate amount of water or an aqueous solution containing 1- to 100-equivalent(s) of the corresponding acid (acetic acid, hydrochloric acid, sulfuric acid, methanesulfonic acid, etc.), followed by heating for 0.5 minute to 20 hours at 40°C to 130°C, preferably 60°C to 70°C. When there is anything which is not completely dissolved in the resulting solution, filtration is if necessary conducted either under ordinary pressure *or in vacuo* to remove the insoluble matters.

To the resulting solution, at 0°C to 130°C, preferably at 60°C to 80°C or, more preferably at 70°C, added is an appropriate amount of a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solution thereof; preferably a solvent selected from the group consisting of methanol, ethanol, 1-propanol, 2-propanol, 1-butanol, 2-butanol and a mixed solvent thereof; or, more preferably, ethanol, followed by stirring at 0°C to 35°C or, preferably at room temperature, for 0.1 to 100 hour(s), preferably for 15 to 20 hours or, more preferably for 18 hours.

The resulting solid is dried at ordinary pressure or *in vacuo* and washed for one to several times with a solvent selected from the group consisting of dimethyl sulfoxide, N,N-dimethylacetamide, N,N-dimethylformamide, 1,2-dimethoxyethane, diethyl ether, tert-butyl methyl ether, acetone, ethyl acetate, hexane, dichloromethane, chloroform, tetrachloromethane, 1,2-dichloroethane and a mixed solvent thereof; preferably a solvent selected from the group consisting of methanol, ethanol, 1-propanol, 2-propanol, 1-butanol, 2-butanol and a mixed solvent thereof; or, more preferably ethanol or 2-propanol.

The resulting solid is dried *in vacuo* at 25°C to 80°C, preferably at 40°C to 60°C or, more preferably at 50°C, for 0.5 to 100 hour(s) or, preferably for 60 to 80 hours or, more preferably for 72 hours, to give the desired crystals of a free base or the desired crystals of intramolecular salt of the compound represented by the formula (I).

Moreover, a pharmaceutical composition containing the compound represented by the above formula (I) in a crystalline form as an active ingredient together with a pharmaceutically acceptable carrier or diluent; an anti-tumor agent containing the compound represented by the above formula (I) in a crystalline form as an active ingredient together with a pharmaceutically acceptable carrier or diluent; and an anti-tumor agent for injection containing the compound represented by the above formula (I) in a crystalline form as an active ingredient together with a pharmaceutically acceptable carrier or diluent can be easily manufactured using a method which is well-known or common among persons skilled in the art of pharmaceutical preparations. For example, there can be mentioned a liquid agent for intravenous injection, a freeze-dried preparation for intravenous injection and a preparation where powder for intravenous injection is charged.

A liquid preparation for intravenous injection can be manufactured by dissolving the compound represented by the above formula (I) in a crystalline form in an appropriate solvent such as distilled water for injection, physiological saline solution, aqueous solution of glucose, alcohols, plant-derived oil such as soybean oil, liquid for intravenous injection (such as aqueous solution of sodium citrate or citric acid) or a mixed solution thereof.

Further, a freeze-dried preparation for intravenous injection can be prepared by dissolving the compound in a crystalline form represented by the above formula (I) alone or together with an appropriate additive into an appropriate solvent, subdividing into vials for injection or the like, and subjecting it to be freeze-dried.

Furthermore, a preparation in which powder for intravenous injection is charged can be prepared by subdividing the compound in a crystalline form represented by the above formula (I)alone or together with an appropriate additive into vials for injection or the like.

With regard to the liquid for intravenous injection, the previously dissolved one may be used as it is or alternatively a form where administration is conducted by dissolving in an appropriate solvent or diluent before use may be adopted. A freeze-dried preparation for intravenous injection and a preparation where powder for intravenous injection is charged is administered by dissolving in an appropriate solvent or diluent before use. An appropriate solvent or diluent used here includes, for example, distilled water for injection, physiological saline solution, aqueous solution of glucose, alcohols, liquid for intravenous injection (such as aqueous solution of sodium citrate and citric acid), an electrolyte solution (for intravenous drip infusion and intravenous injection) or a mixed solution thereof.

Now, with regard to thermostability, light stability, solubility and hygroscopicity, data of the compound represented by the formula (I) in a crystalline form and the compound represented by the formula in an amorphous form are compared.

### (1) Thermostability test

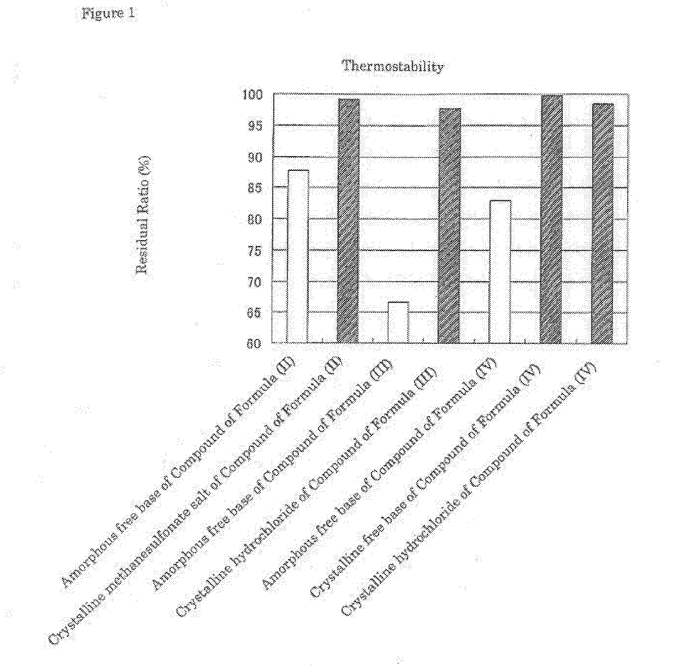

A solid thermostability test of amorphous free bases and crystals of the compounds represented by the formula (II), the formula (III) and the formula (IV) is shown in Table 1. This test is an accelerated test conducted at 80°C in tightly closed vials. As shown in Table 1, the crystals show a significantly excellent thermostability as compared with the corresponding amorphous substances (Fig. 1).

**Table 1 Comparative Data for Thermostability of Solid (Residual rate is the data after 4 weeks)**

| Samples | Residual Rate (%) |
|---|---|
| Amorphous free base of the compound of the formula (II) | 87.8 |
| Crystals of methanesulfonate of the compound of the formula (II) | 99.1 |
| Amorphous free base of the compound of the formula (III) | 66.6 |
| Crystals of hydrochloride of the compound of the formula (III) | 97.7 |
| Amorphous free base of the compound of the formula (IV) | 82.9 |
| Crystals of free base of the compound of the formula (IV) | 99.7 |
| Crystals of hydrochloride of the compound of the formula (IV) | 98.4 |

### (2) Light stability test

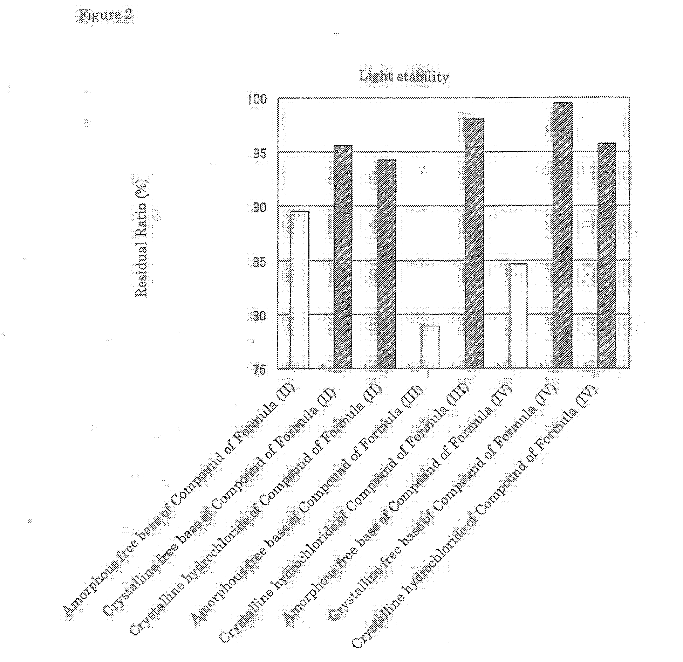

A solid light stability test of amorphous free bases and crystals of the compounds represented by the formula (II), the formula (III) and the formula (IV) is shown in Table 2. This test was carried out at 25°C under the light source of 2000 luxes in tight-closed vials. As shown in Table 2, the crystals show a significantly excellent light stability as compared with the corresponding amorphous substances (Fig. 2).

**Table 2 Comparative Data for Light Stability of Solid (Residual rate is the data after 4 weeks)**

| Samples | Residual Rate (%) |
|---|---|
| Amorphous free base of the compound of the formula (II) | 89.5 |
| Crystals of free base of the compound of the formula (II) | 95.6 |
| Crystals of hydrochloride of the compound of the formula (II) | 94.3 |
| Amorphous free base of the compound of the formula (III) | 78.9 |
| Crystals of hydrochloride of the compound of the formula (III) | 98.1 |
| Amorphous free base of the compound of the formula (IV) | 84.6 |
| Crystals of free base of the compound of the formula (IV) | 99.5 |
| Crystals of hydrochloride of the compound of the formula (IV) | 95.8 |

### (3) Test on solubility in water

Solubilities of crystals of intramolecular salts of the compounds of the formulae (II), (III) and (IV) and the corresponding amorphous free bases in water are shown below.

Amorphous free base of the compound represented by the formula (II): not more than 0.001 mg/mL

Crystals of hydrochloride of the compound represented by the formula (II): 10 to 50 mg/mL

Crystals of methanesulfonate of the compound represented by the formula (II): 10 to 50 mg/mL

Amorphous free base of the compound represented by the formula (III): not more than 0.001 mg/mL

Crystals of hydrochloride of the compound represented by the formula (III): 50 to 100 mg/mL

Amorphous free base of the compound represented by the formula (IV): not more than 0.001 mg/mL

Crystals of hydrochloride of the compound represented by the formula (IV): 10 to 50 mg/mL

From the above, it can be said that each of the intramolecular salts of the compounds represented by the formulae (II), (III) and (IV) shows a significantly excellent solubility in water as compared with each of the corresponding amorphous free bases.

### (4) Hygroscopicity test

Comparing the hygroscopicity of amorphous free base of the compound represented by the above formula (II) with that of crystals of hydrochloride of the same compound, the weight of the amorphous free base increased by 7.6% under the moisturized condition (adsorption process of water), while the weight of the crystal increased as slightly as 1.5% in the case where the comparison was done changing the humidity from 30% to 75% which is the humidity for usual preservation. Thus, the weight change rate of the amorphous free base of the compound represented by the formula (II) upon humidification (relative humidity changes from 30% to 75%) was 7.6%. On the other hand, the weight change rate of the crystals of hydrochloride of the compound represented by the formula (II) upon humidification (relative humidity changes from 30% to 75%) was 1.5%, therefore, the influence by humidification was very little.

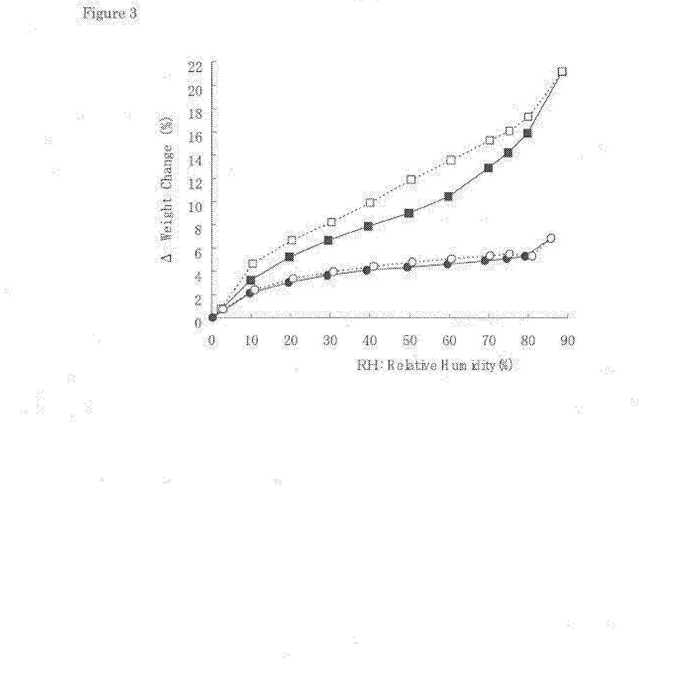

The changes in weight under the dehumidifying condition (desorption process of water) are greatly different from changes in weight under the humidifying condition in the case of the amorphous free base; therefore, dissolution due to hygroscopicity, etc. may be suspected. However, in the case of crystals, the changes in weight were nearly the same under both conditions, so little influence by absorption of moisture was observed (refer to Fig. 3).

Further, comparing hygroscopicity of the amorphous free base of the compound of the above formula (IV) with that of crystals of the same compound, the weight of the amorphous free base increased by 2.8%, while the weight of the crystal increased as slightly as 0.6% in the case where the comparison was done changing the humidity from 30% to 75%. Thus, the weight change rate of the amorphous free base of the compound represented by the formula (IV) upon humidification (relative humidity of 30% to 75%) was 2.8%. On the other hand, the weight change rate of the crystals of hydrochloride of the compound represented by the formula (IV) upon humidification (relative humidity of 30% to 75%) was 0.6%, therefore, the influence by humidification was very little.

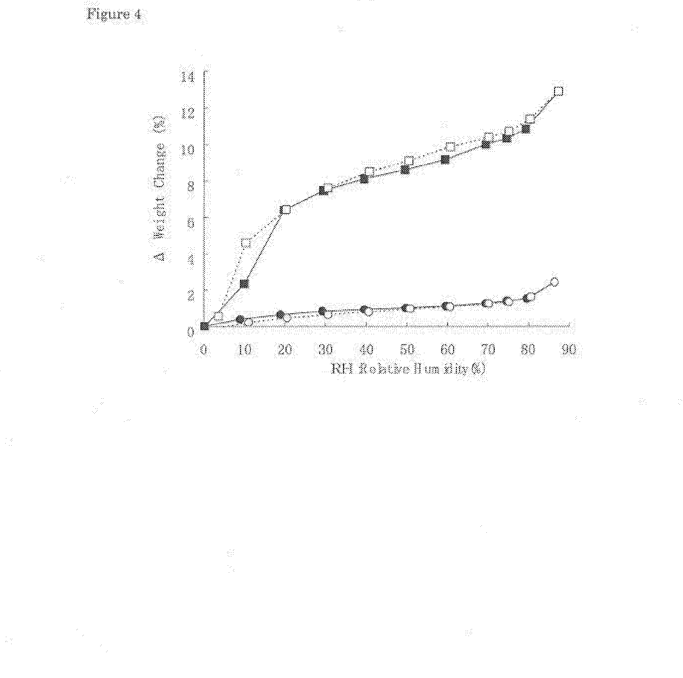

Furthermore, the changes in weight under the dehumidifying condition are significantly different from the changes in weight under the humidifying condition in the case of the amorphous free base and, therefore, dissolution due to hygroscopicity, etc. may be suspected. However, in the case of crystals, the changes in weight were nearly the same under both conditions, no influence by absorption of moisture being not observed (refer to Fig. 4). In other words, it can be said that making crystals leads to significantly improved hygroscopicity of the amorphous substance.

It is recognized from the above that the compound in a crystalline form represented by the formula (I), particularly, the free base and the intramolecular salt of the compound in a crystalline form represented by the formula (II); the free base and the intramolecular salt of the compound in a crystalline form represented by the formula (III); and the free base and the intramolecular salt of the compound in a crystalline form represented by the formula (IV) show significantly excellent thermostability, light stability, water solubility and low hygroscopicity as compared with the corresponding substances in a non-crystalline form (amorphous free bases). Moreover, as shown in the Reference Example, crystallization of the amorphous free base can lead to greatly improved purity of the compound represented by the formula (I).

### Brief Description of the Drawings

Fig. 1 is a drawing which shows the results of the solid thermostability test (an acceleration test at 80°C) of the amorphous free bases of the compound represented by the formula (II), the formula (III) and the formula (IV) and the crystals thereof. Open columns in the drawing show the residual rate (%) of the amorphous samples after 4 weeks, while columns with oblique lines shows the residual rate (%) of the crystalline samples.
Fig. 2 shows the results of the solid light stability test (test at 25°C under a 2000-lux light source) of the amorphous free bases of the compound represented by the formula (II), the formula (III) and the formula (IV), and the crystals of each of them. Open columns in the drawing show the residual rate (%) of the amorphous samples after 4 weeks, while columns with oblique lines shows the residual rate (%) of the crystalline samples.
Fig. 3 is a drawing which shows the results of hygroscopicity test of the amorphous free base of the compound represented by the above formula (II) and the crystals of hydrochloride of the same compound.

The symbol ■ in the drawing shows the weight change rates (%) of the amorphous free base of the compound represented by the formula (II) upon humidification, while the symbol □ shows the weight change rates (%) of the amorphous free base of the same compound upon desorption. On the other hand, The symbol • in the drawing shows weight change rates (%) of the hydrochloride crystals of the compound represented by the formula (II) upon humidification, while the symbol ○ shows the weight change rates (%) of the hydrochloride crystals of the same compound upon desorption.

Fig. 4 is a drawing which shows the results of a hygroscopicity test of the amorphous free base of the compound represented by the above formula (IV) and the hydrochloride crystals of the same compound.

The symbol ■ in the drawing shows the changes in weight (%) of the amorphous free base of the compound represented by the formula (IV) upon humidification, while the symbol □ shows the weight change rates (%) of the amorphous free base of the same compound upon dehumidification. On the other hand, the symbol • in the drawing shows the weight change rates (%) of the hydrochloride crystals of the compound represented by the formula (IV) upon humidification, while the symbol ○ shows weight change rates (%) of the hydrochloride crystals of the same compound upon dehumidification.

Fig. 5 is a powder X-ray diffraction pattern of crystals of the compound 2 as produced by the process mentioned in Example 2-1.

Fig. 6 is a picture (under a polarization microscope; 400 magnifications) of crystals of the compound 3 as produced by the process mentioned in Example 3.

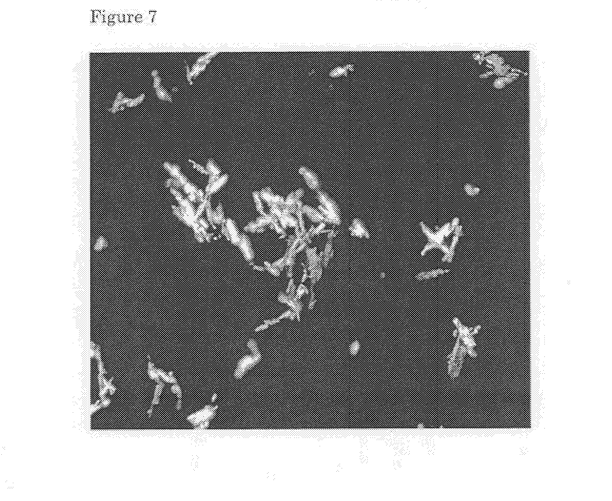

Fig. 7 is a picture (under a polarization microscope; 400 magnifications) of crystals of the compound 4 as produced by the process mentioned in Example 4.

Fig. 8 is a powder X-ray diffraction pattern of crystals of the compound 8 as produced by the process mentioned in Example 7-1.

### Best Mode for Carrying Out the Invention

The present invention will now be illustrated more specifically by way of the following Examples although it goes without saying that the present invention is not limited to those Examples only.

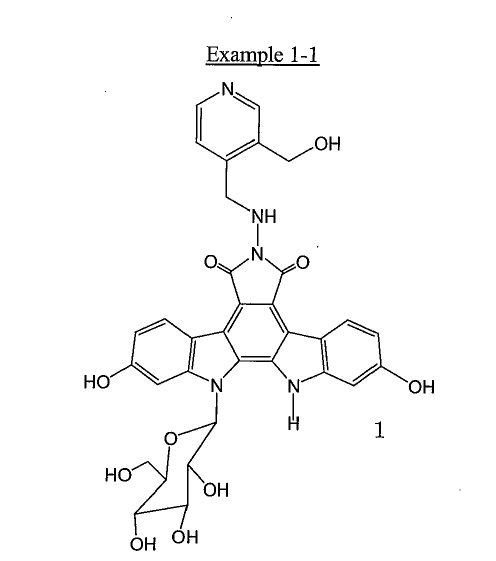

Process A for crystals of the compound 1 (according to the above-mentioned Manufacturing Method 2):A non-crystalline compound 1 (500 mg) prepared by a process mentioned in Example 14 of the specification of JP-A-10-245390 was added to acetic acid (0.218 ml) and methanol (500 ml), followed by heating to reflux for 15 hours. After dissolving the compound 1 completely, concentration was conducted by evaporating 400 ml of methanol under ordinary pressure, and a suspension *per se* of the separated solid was heated to reflux for 15 hours. After it was cooled down to room temperature, the resulting solid was filtered, washed for three times with methanol (1 ml) and dried *in vacuo* at 40°C for 24 hours to give 381 mg of a red solid. The resulting red solid (250 mg) was suspended in ethanol (6.5 ml) and heated with stirring at 75°C for 20 hours to give yellow crystals. After cooling down to room temperature, the resulting crystals were filtered, washed with ethanol (1 ml) three times and dried *in vacuo* at 50°C for 72 hours to give a yellow crystal compound 1 (247 mg).

### Molecular weight:

ESI (m/z): 654 (M-H)⁻

### Proton nuclear magnetic resonance (NMR) spectral data:

¹H-NMR(400 MHz, d-dimethyl sulfoxide(DMSO-d6), δppm):11.17(1H, s), 9.79(2H, s), 8.84(1H, d, J=8.8Hz), 8.75(1H, d, J=8.4Hz), 8.50(1H, s), 8.40(1H, d, J=5.1Hz), 7.59(1H, d, J=5.1Hz), 7.17(1H, s), 6.97(1H, s), 6.82(1H, d, J=8.8Hz), 6.80(1H, d, J=8.8Hz), 6.26(1H, t, J=4.4Hz), 5.96(1H, d, J=8.1Hz), 5.88(1H, s), 5.39(1H, s), 5.24(1H, s), 5.14(1H, s), 4.93(1H, d, J=4.0Hz), 4.74(2H, s), 4.36(2H, d, J=4.4Hz), 3.76-4.02(4H, m), 3.43-3.52(2H, m)

### Infrared absorption (IR) spectra:

IR (ATR: Attenuated Total Reflection Infrared): 3315, 1746, 1697, 1620, 1576, 1458, 1371, 1333, 1232, 1198, 1119, 1069, 1015, 615 cm⁻¹

### High-performance liquid chromatography (HPLC) purity data:

Purity: 99.6% (Reversed phase column: YMC-Pack Pro C18, AS-303)

**Table 3**

| 2θ | Diffraction intensity (cps) |
|---|---|
| 4.4 | 30 |
| 7.3 | 21 |
| 7.6 | 100 |
| 8.9 | 27 |
| 10.8 | 15 |
| 12.7 | 16 |
| 17.2 | 19 |
| 17.4 | 28 |
| 17.8 | 30 |
| 19.6 | 26 |
| 19.7 | 21 |
| 20.6 | 34 |
| 20.8 | 23 |
| 21.0 | 17 |
| 21.5 | 31 |
| 23.2 | 26 |
| 24.1 | 34 |
| 24.4 | 36 |
| 24.7 | 26 |
| 25.0 | 21 |
| 25.2 | 19 |
| 25.5 | 33 |
| 25.7 | 30 |
| 26.3 | 49 |
| 27.1 | 39 |
| 27.1 | 44 |
| 28.3 | 16 |
| 30.4 | 15 |
| 30.5 | 15 |
| 32.0 | 14 |

| | |
|---|---|
| Note: Each intensity is a relative value when the maximum value is 100. | |

The above powder X-ray diffraction analysis data were measured by an automatic X-ray Rint-Ultima + system (2 kW) (manufactured by Rigaku International Corporation). The analytical method is as follows.
X-ray radiation source: copper (Cu)
Tube voltage/tube current: 40 kV/30 mA
Monochromator: Automatic monochromator
Goniometer: Wide-angle goniometer
Scan step: 0.02°(deg.)
Scan speed: 2.00°(deg.)/minute
Divergence slit: 1° (deg.)
Scattering slit: 1° (deg.)
Receiving slit: 0.15 mm
Measuring temperature: room temperature

### Measurement of residual solvent:

Ethanol < 0.01%

The content of the residual solvent was measured by a head space sampler/gas chromatograph/mass selective detector (HSS/GC/MSD) (manufactured by Agillent Technologies).

### Hygroscopicity test:

Hygroscopicity test was conducted by IGAsorp Moisture Sorption Analyzer, manufacture by Hiden Analytical Limited (refer to Fig. 3).

### Example 1-2

Process B for crystals of the compound 1 (according to the above-mentioned Manufacturing Process 4): A non-crystalline compound 1 (30 mg) prepared by a process mentioned in Example 14 of the specification of JP-A-10-245390 was suspended in ethanol (1.0 ml) and acetic acid (0.005 ml) and heated with stirring at 75°C for 15 hours to give yellow crystals. After the resulting solution was cooled down to room temperature, the crystals were filtered, washed three times with ethanol (1 ml) and dried *in vacuo* at 50°C for 120 hours to give 29 mg of a yellow crystal compound 1.

The crystals obtained by the process shown in Example 1-2 also showed the same powder X-ray diffraction spectra as those for Example 1-1. 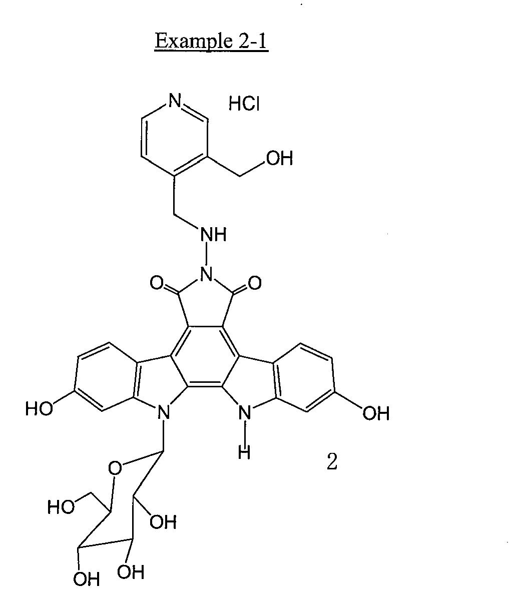 Process A for crystals of the compound 2 (according to the above-mentioned Manufacturing Method 1): A non-crystalline compound 1 (500 mg) prepared by a process mentioned in Example 14 of the specification of JP-A-10-245390 was added to a mixed solvent comprising methanol (300 ml) and a hydrochloric acid-methanol reagent (10 ml), followed by heating to reflux for 4 hours. After dissolving the compound 1 completely, concentration was conducted by evaporating 200 ml of methanol under ordinary pressure, and a suspension *per se* of the separated solid was heated to reflux for 15 hours. After it was cooled down to room temperature, the resulting solid was filtered, washed three times with methanol (1 ml) and dried *in vacuo* at 40°C for 24 hours to give 393 mg of a yellow solid. The resulting yellow solid (240 mg) as such was suspended in ethanol (6 ml) and heated with stirring at 75°C for 20 hours. After cooling down to room temperature, the yellow crystals were filtered, washed with ethanol (1 ml) three times and dried *in vacuo* at 50°C for 72 hours to give a yellow crystal compound 2 (235 mg).

ESI (m/z): 654 (M-H)⁻
¹H-NMR(400 MHz, DMSO-d6, δppm):11.18(1H, s), 9.82(2H, s), 8.83(2H, m), 8.76 (1H, d, J=8.4Hz), 8.71 (1H, s), 8.51 (1H, d, J=5.9Hz), 7.17 (1H, d, J=1.8Hz), 6.97 (1H, d, J=2.2Hz), 6.83(1H, dd, J=8.4, 1.8Hz), 6.81 (1H, dd, J=8.4,2.2Hz), 5.97(1H, d, J=8.8Hz), 4.83(2H, s), 4.58(2H, s), 3.72-4.03(4H, m), 3.40 -3.55 (2H, m)
IR (ATR): 3240, 1738, 1693, 1620, 1576, 1472, 1410, 1377, 1339, 1321, 1225, 1186, 1121, 1051, 1005, 905, 829, 818, 743 cm⁻¹
Purity: 99.1 %

The above purity data were measured under the same condition as in Example 1-1.

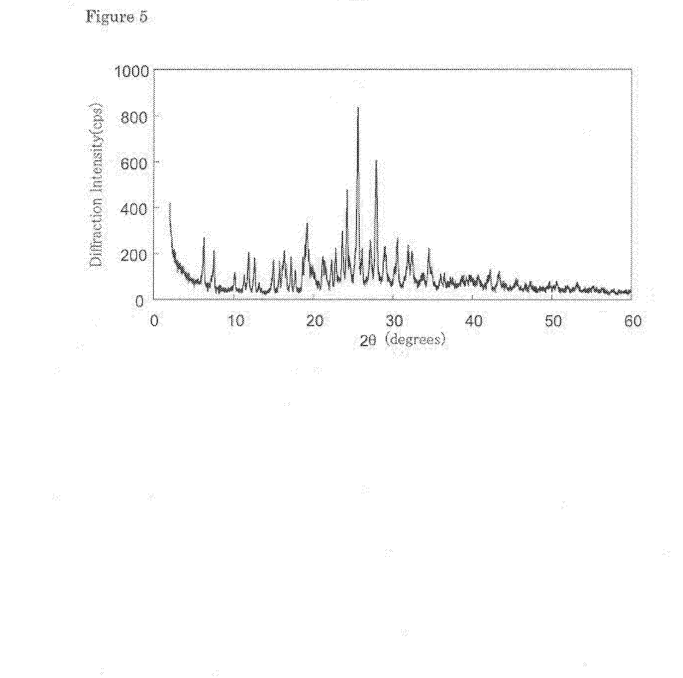

**Table 4 Powder X-Ray Diffraction Data (refer to Fig. 5)**

| 2θ | Diffraction intensity (cps) |
|---|---|
| 6.3 | 32 |
| 7.5 | 25 |
| 11.9 | 24 |
| 12.6 | 20 |
| 15.0 | 21 |
| 15.7 | 20 |
| 16.3 | 24 |
| 16.6 | 19 |
| 17.2 | 22 |
| 18.7 | 19 |
| 19.0 | 27 |
| 19.2 | 40 |
| 19.5 | 23 |
| 21.2 | 22 |
| 21.4 | 18 |
| 22.4 | 21 |
| 22.8 | 27 |
| 23.7 | 35 |
| 24.3 | 57 |
| 25.6 | 100 |
| 26.2 | 20 |
| 27.2 | 31 |
| 28.0 | 67 |
| 28.9 | 21 |
| 29.0 | 26 |
| 29.2 | 23 |
| 30.6 | 30 |
| 31.9 | 24 |
| 32.5 | 23 |
| 34.6 | 27 |

| | |
|---|---|
| Note: Each intensity is a relative value when the maximum value is 100. | |

The above powder X-ray diffraction analysis data were measured under the same condition as in Example 1-1.

### Quantitative determination of ion:

Hydrochloric acid (HCl) 1.01 molar ratio

Quantitative determination of ion was conducted by capillary electrophoresis (HPCE; manufactured by Agilent Technologies).

### Residual solvent: Ethanol (EtOH) < 0.01%

The above analytical data for residual solvent were measured under the same condition as in Example 1-1.

### Example 2-2

Process B for crystals of the compound 2 (according to the above-mentioned Manufacturing Method 3): A non-crystalline compound 1 (30 mg) prepared by a process mentioned in Example 14 of the specification of JP-A-10-245390 was suspended in ethanol (1.0 ml) and a 1 molar concentration (M) aqueous solution of hydrochloric acid (0.09 ml), and heated with stirring at 75°C for 15 hours to give yellow crystals. After the resulting solution was cooled down to room temperature, the crystals were filtered, washed three times with ethanol (1 ml) and dried *in vacuo* at 50°C for 24 hours to give a yellow crystal compound 2 (29 mg).

The crystals obtained by the process shown in Example 2-2 also showed the same powder X-ray diffraction spectra as those for Example 2-1.

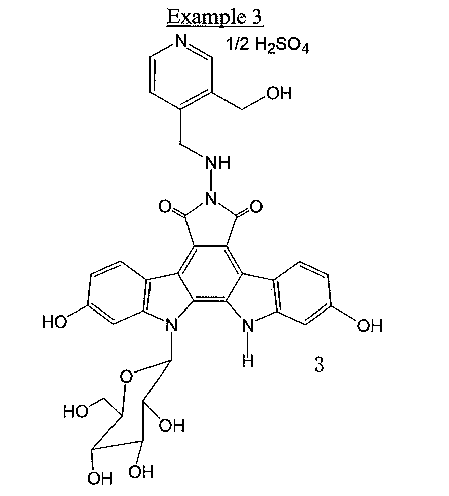

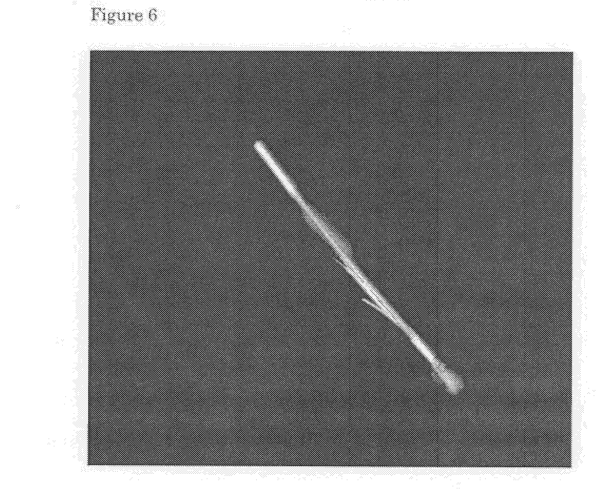

Process for crystals of the compound 3 (according to the above Manufacturing Method 5): A non-crystalline compound 1 (500 mg) prepared by a process mentioned in Example 14 of specification of JP-A-10-245390 was added to a mixed solvent comprising ethanol (180 ml), water (180 ml) and sulfuric acid (0.0407 ml) and was dissolved therewith by heating at 70°C with stirring. When the solution was slowly stirred at room temperature for 18 hours to give orange-colored crystals. The crystals were filtered, washed with ethanol (1 ml) three times and dried *in vacuo* at 50°C for 72 hours to give an orange-colored needle compound 3 (491 mg) (refer to Fig. 6).

ESI (m/z); 654 (M-H)⁻
¹H-NMR(400 MHz, DMSO-d6, δppm):11.19(1H, s), 9.80(1H, s), 9.77 (1H, s), 8.84(1H, d, J=8.8Hz), 8.78 (1H, d, J=8.8Hz), 8.63 (1H, d, J=5.5Hz), 8.62(1H, s), 8.08 (1H, d, J=5.5Hz), 7.17 (1H, d, J=1.1Hz), 6.97 (1H, d, J=1.8Hz), 6.83(1H, dd, J=8.8 , 1.1Hz), 6.80 (1H, dd, J=8.8,1.8Hz), 6.41(1H, bs), 5.97(1H, d, J=8.8Hz), 5.87(1H, bs), 5.05-5.72(3H, m), 4.92 (1H, bs), 4.79(2H, s), 4.48(2H, s), 3.76-4.03(4H, m), 3.41-3.54 (2H, m)
IR (ATR): 3290, 2360, 1744, 1692, 1618, 1578, 1458, 1400, 1375, 1329, 1231, 1196, 1120, 1049, 1015, 739 cm⁻¹
Purity: 98.4%

The above purity data were measured under the same condition as in Example 1-1.

### Quantitative determination of ion:

H₂SO₄ 0.48 molar ratio

The above data for quantitative analysis of ion was measured under the same condition as in Example 2-1.

Residual solvent: no ethanol was detected.

The above data for residual solvent analysis was measured under the same condition as in Example 1-1.

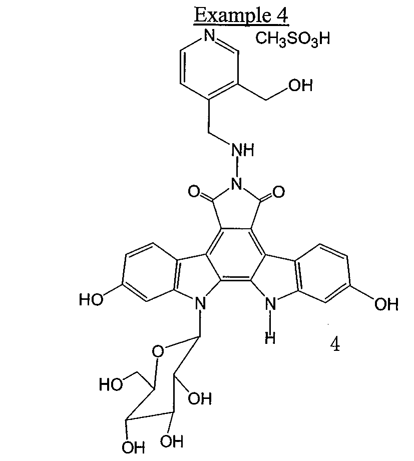

Process for crystals of the compound 4 (according to the above Manufacturing Method 5): A non-crystalline compound 1 (50 mg) prepared by a process mentioned in Example 14 of specification of JP-A-10-245390 was added to a mixed solvent comprising ethanol (1.8 ml), water (0.5 ml) and methanesulfonic acid (0.0099 ml) and was dissolved therewith by heating at 65°C with stirring. The solution was slowly stirred at room temperature for 22 hours to give yellowish orange-colored crystals. The crystals were filtered, washed with ethanol (1 ml) three times and dried *in vacuo* at 50°C for 72 hours to give a yellowish orange-colored needle compound 4 (49 mg) (refer to Fig. 6).

ESI (m/z); 654 (M-H)⁻
¹H-NMR(400 MHz, DMSO-d6, δppm):11.18(1H, s), 9.80(1H, s), 9.77(1H, s), 8.84(1H, d, J=8.4Hz), 8.81(1H, d, J=5.5Hz), 8.76(1H, d, J=8.4Hz), 8.71(1H, s), 8.46(1H, d, J=5.5Hz), 7.17(1H, s), 6.97(1H, s), 6.82(1H, d, J=8.4Hz), 6.79(1H, d, J=8.4Hz), 6.52(1H, bs), 5.97(1H, d, J=9.5Hz), 5.83(1H, bs), 4.83(2H, s), 4.56(2H, s), 3.75-4.02(4H, m), 3.40-3.53(2H, m), 2.30(3H, s)
IR (ATR): 3311, 1747, 1699, 1620, 1583, 1456, 1329, 1196, 1153, 1124, 1042, 1016, 779, 742 cm⁻¹

### Quantitative determination of ion:

Methanesulfonic acid (CH₃SO₃H) 1.00 molar ratio

The above data for quantitative analysis of ion was measured under the same condition as in Example 2-1.

Residual solvent: no ethanol was detected.

The above data for residual solvent analysis was measured under the same condition as in Example 1-1. 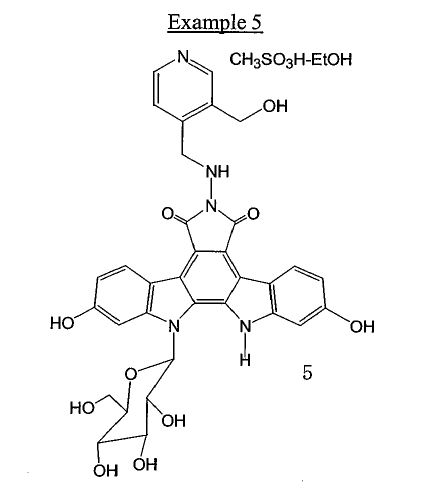 Process for crystals of the compound 5 (according to the above-mentioned Manufacturing Method 3): A non-crystalline compound 1 (109 mg) prepared by a process mentioned in Example 14 of the specification of JP-A-10-245390 was suspended in ethanol (3.6 ml) and methanesulfonic acid (0.022 ml) and heated with stirring at 75°C for 15 hours to give yellow crystals. After the resulting solution was cooled down to room temperature, the crystals were filtered, washed three times with ethanol (1 ml) and dried *in vacuo* at 50°C for 24 hours to give a yellow crystal compound 5 (107 mg).

ESI (m/z): 654 (M-H)⁻
¹H-NMR(400 MHz, DMSO-d6, δppm):11.18(1H, s), 9.77(2H, bs), 8.83(1H, d, J=8.8Hz), 8.83(1H, d, J=5.9Hz), 8.86 (1H, d, J=8.8Hz), 8.71(1H, s), 8.50 (1H, d, J=5.9Hz), 7.17(1H, s), 6.97(1H, s), 6.82(1H, d, J=8.8Hz), 6.80(1H, d, J=8.8Hz), 5.97(1H, d, J=8.4Hz), 4.83(2H, s), 4.57(2H, s), 3.75-4.03(4H, m), 3.42-3.52(4H, m), 2.30(3H, s), 1.05(3H, t, J=7.0Hz)
IR (ATR): 3312, 2341, 1753, 1707, 1622, 1581, 1456, 1377, 1327, 1232, 1196, 1111, 1022, 745, 635, 617 cm¹
Purity: 99.2%

The above purity data were measured under the same condition as in Example 1-1.

**Table 5 Powder X-Ray Diffraction Data**

| 2θ | Diffraction intensity (cps) |
|---|---|
| 7.9 | 20 |
| 8.1 | 21 |
| 12.7 | 20 |
| 13.0 | 26 |
| 16.0 | 14 |
| 16.9 | 28 |
| 17.6 | 41 |
| 18.1 | 30 |
| 18.6 | 18 |
| 19.2 | 13 |
| 20.3 | 11 |
| 21.3 | 13 |
| 21.4 | 16 |
| 22.2 | 15 |
| 23.3 | 47 |
| 24.5 | 15 |
| 24.8 | 19 |
| 25.6 | 20 |
| 26.2 | 93 |
| 26.3 | 100 |
| 26.9 | 18 |
| 28.2 | 11 |
| 28.3 | 14 |
| 28.5 | 16 |
| 29.0 | 10 |
| 29.2 | 10 |
| 32.3 | 11 |
| 35.4 | 11 |
| 38.9 | 12 |
| 39.0 | 10 |

| | |
|---|---|
| Note: Each intensity is a relative value when the maximum value is 100. | |

The above powder X-ray diffraction analysis data were measured under the same condition as in Example 1-1.

### Quantitative determination of ion:

Methanesulfonic acid 1.14 molar ratio

The above data for quantitative analysis of ion was measured under the same condition as in Example 2-1.

Residual solvent: ethanol 5.49%

The above data for residual solvent analysis was measured under the same condition as in Example 1-1.

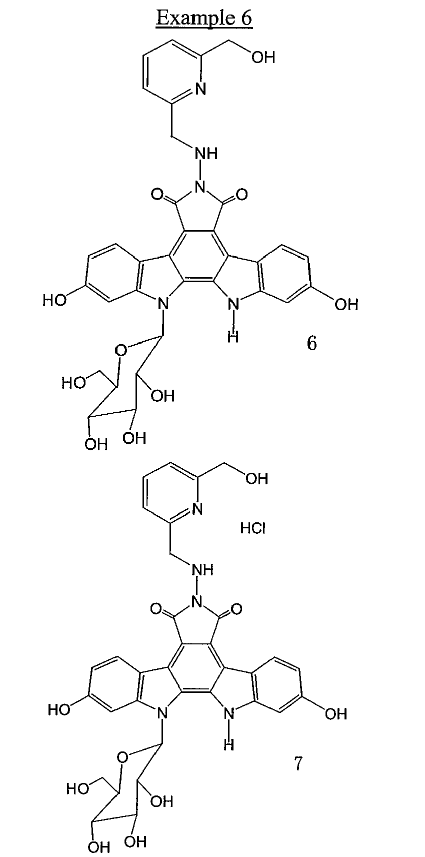

Process for crystals of the compound 7 (according to the above Manufacturing Method 3): A non-crystalline compound 6 (500 mg) prepared by a process mentioned in Example 27 of the specification of JP-A-10-245390 was suspended in ethanol (15 ml) and 6M aqueous hydrochloric acid (0.25 ml) and heated with stirring at 75°C for 168 hours to give orange-colored crystals. After the resulting solution was cooled down to room temperature, the crystals were filtered, washed for three times with ethanol (1 ml) and dried *in vacuo* at 50°C for 72 hours to give the compound 7 (430 mg) in orange-colored crystals.
ESI (m/z): 654 (M-H)⁻
¹H-NMR(400 MHz, DMSO-d6, δppm):11.18(1H, s), 9.82(2H, s), 8.80(1H, d, J=8.8Hz), 8.73(1H, d, J=8.8Hz), 8.38 (1H, t, J=7.3Hz), 8.13(1H, d, J=7.3Hz), 7.77(1H, d, J=7.7Hz), 7.17 (1H, s), 6.97 (1H, s), 6.83(1H, d, J=8.8Hz), 6.80 (1H, d, J=8.8Hz), 5.97(1H, d, J=8.5Hz), 4.77 (2H, s), 4.63 (2H, s), 3.75-4.03(4H, m), 3.42-3.54(2H, m)
IR (ATR): 3170, 1738, 1688, 1614, 1580, 1415, 1345, 1290, 1250, 1198, 1088, 1061, 1002, 907, 831, 799, 741 cm⁻¹
Purity: 99.5%

The above purity data were measured under the same condition as in Example 1-1.

**Table 6 Powder X-Ray Diffraction Data**

| 2θ | Diffraction intensity (cps) |
|---|---|
| 6.5 | 54 |
| 6.7 | 57 |
| 9.3 | 12 |
| 11.9 | 15 |
| 13.4 | 18 |
| 15.9 | 14 |
| 16.3 | 12 |
| 16.9 | 14 |
| 17.3 | 42 |
| 17.8 | 36 |
| 18.0 | 19 |
| 19.4 | 14 |
| 20.0 | 19 |
| 20.1 | 17 |
| 22.1 | 13 |
| 23.6 | 22 |
| 24.2 | 85 |
| 24.7 | 100 |
| 25.5 | 93 |
| 25.7 | 69 |
| 26.7 | 21 |
| 27.1 | 25 |
| 27.5 | 14 |
| 28.1 | 18 |
| 28.4 | 14 |
| 28.8 | 17 |
| 30.6 | 12 |
| 33.1 | 14 |
| 34.8 | 15 |
| 35.0 | 15 |

| | |
|---|---|
| Note: Each intensity is a relative value when the maximum value is 100. | |

The above powder X-ray diffraction analysis data were measured under the same condition as in Example 1-1.

### Quantitative determination of ion:

Hydrochloric acid 1.13 molar ratio

The above data for quantitative analysis of ion was measured under the same condition as in Example 2-1.

### Residual solvent: ethanol 0.27%

The above data for residual solvent analysis was measured under the same condition as in Example 1-1.

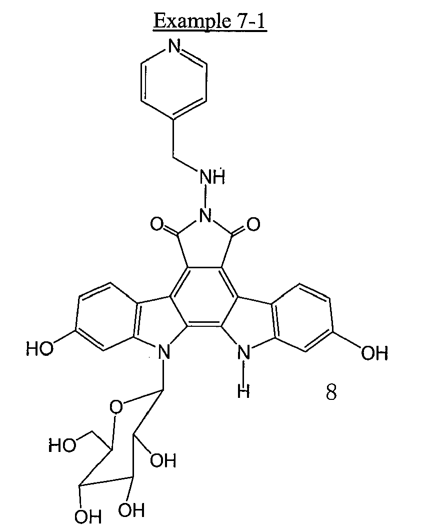

Process for crystals of the compound 8:

### 1) Process for production of the non-crystalline compound 8

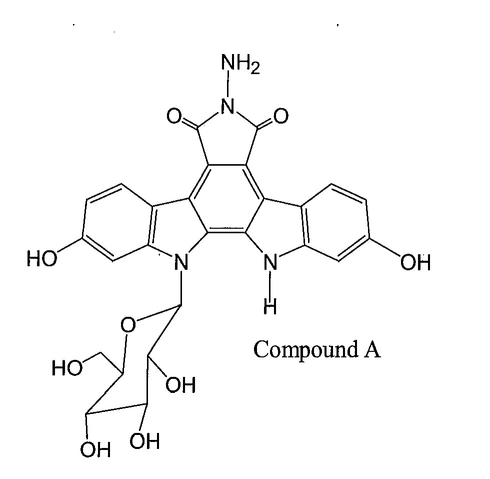

The compound A (a hydrazine substance) (6.3 g) and 45 ml of 4-pyridinecarboaldehyde were dissolved in 210 ml of methanol and, after 1.76 ml of acetic acid were added, the mixture was stirred overnight at 70°C. The solid separated out therefrom was filtered and washed with methanol, the resulting solid was dissolved in 7.0 L of a mixed solvent of methanol-tetrahydrofuran (1:1) and 1 g of 10% palladium-carbon was added thereto, followed by stirring overnight in a hydrogen atmosphere. The resulting reaction solution was filtered through Celite and the filtrate was concentrated. The residue was filled in a Sephadex LH-20 column chromatography and developed with methanol. The desired fraction was concentrated and dried to give a compound 8 (3.3 g).

### 2) Process for production of crystals of the compound 8 (according to the above Manufacturing Method 2)

A non-crystalline compound 8 (250 mg) prepared in the above 1) was added to methanol (500 ml) and acetic acid (0.135 ml) followed by heating to reflux for 15 minutes. After dissolving the compound 8 completely, concentration was conducted by evaporating 430 ml of methanol from the resulting solution under ordinary pressure and a suspension *per se* of the separated solid was heated to reflux for 2.5 hours. After the solution was cooled down to room temperature, the resulting solid was filtered, washed three times with methanol (1 ml) and dried *in vacuo* at 50°C for 2 hours to give 200 mg of a yellow solid. The resulting yellow solid (200 mg) prepared as such was suspended in ethanol (5 ml) and heated with stirring at 75°C for 20 hours. After the resulting solution was cooled down to room temperature, the yellow crystals were filtered, washed with ethanol (1 ml) three times and dried *in vacuo* at 50°C for 72 hours to give a yellow crystal compound 8 (197 mg).
ESI (m/z): 624 (M-H)⁻
¹H-NMR(400 MHz, DMSO-d6, δppm):11.17(1H, s), 9.78(1H, s), 9.76(1H, s), 8.84(1H, d, J=8.8Hz), 8.76(1H, d, J=8.8Hz), 8.47(2H, d, J=5.5Hz), 7.54(2H, d, J=5.5Hz), 7.16(1H, d, J=1.8Hz), 6.96(1H, d, J=1.8Hz), 6.82(1H, dd, J=8.8, 1.8Hz), 6.80(1H, dd, J=8.8,1.8Hz), 6.32(1H, t, J=3.7Hz), 5.95(1H, d, J=8.4Hz), 5.87(1H, t, J=3.7Hz), 5.34 (1H, d, J=3.7Hz), 5.12 (1H, d, J=4.8Hz), 4.91(1H, d, J=4.8Hz), 4.33(2H, d, J=4.4Hz), 3.76-4.02(4H, m), 3.44-3.52(2H, m)
IR (ATR): 1749, 1690, 1630, 1578, 1456, 1400, 1369, 1329, 1253, 1196, 1153, 1121, 1080, 1061, 1013, 800, 743, 617 cm⁻¹
Purity: 98.2%

The above purity data were measured under the same condition as in Example 1-1.

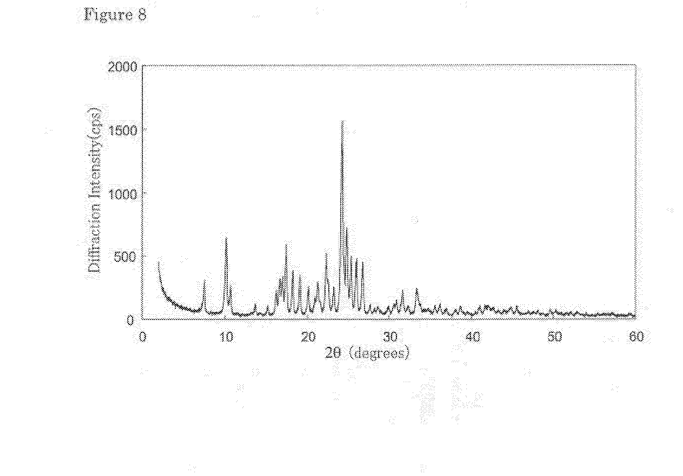

**Table 7 Powder X-Ray Diffraction Data; refer to Fig. 8**

| 2θ | Diffraction intensity (cps) |
|---|---|
| 7.6 | 20 |
| 10.2 | 41 |
| 10.7 | 17 |
| 13.6 | 7 |
| 15.2 | 7 |
| 16.2 | 15 |
| 16.7 | 20 |
| 17.0 | 21 |
| 17.4 | 38 |
| 18.2 | 23 |
| 19.1 | 22 |
| 20.2 | 15 |
| 21.0 | 11 |
| 21.3 | 17 |
| 21.6 | 9 |
| 22.3 | 33 |
| 22.6 | 18 |
| 23.2 | 16 |
| 24.2 | 100 |
| 24.8 | 46 |
| 25.4 | 30 |
| 26.0 | 29 |
| 26.8 | 29 |
| 30.6 | 8 |
| 31.0 | 8 |
| 31.7 | 14 |
| 33.4 | 15 |
| 33.8 | 8 |
| 36.2 | 8 |
| 41.6 | 7 |

| | |
|---|---|
| Note: Each intensity is a relative value when the maximum value is 100. | |

The above powder X-ray diffraction analysis data were measured under the same condition as in Example 1-1.
Residual solvent: ethanol 0.10%; methanol 0.32%

The above data for residual solvent analysis was measured under the same condition as in Example 1-1.

### Example 7-2

Process for the compound 8 (according to the above-mentioned Manufacturing Method 4): A non-crystalline compound 8 (30 mg) prepared by a process mentioned in 1) of Example 7 was suspended in ethanol (1 ml) and acetic acid (0.005 ml), and heated with stirring at 75°C for 15 hours to give yellow crystals. After the resulting solution was cooled down to room temperature, the crystals were filtered, washed three times with ethanol (1 ml) and dried *in vacuo* at 50°C for 15 hours to give 27 mg of a yellow crystal compound 8.

The crystals obtained by the process shown in Example 7-2 also showed the same powder X-ray diffraction spectra as those for Example 7-1. 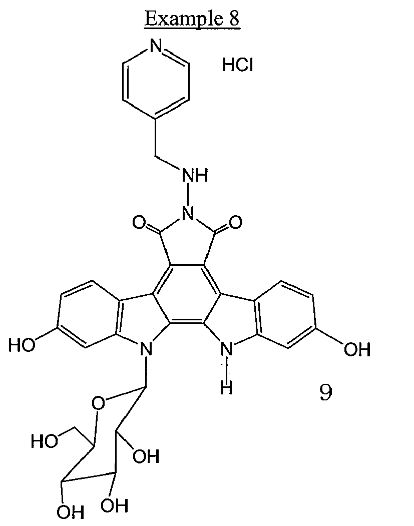 Process for production of crystals of the compound 9 (according to the above Manufacturing Method 3): A non-crystalline compound 8 (300 mg) prepared by a process mentioned in 1) of Example 7 was suspended in ethanol (10 ml) and 1M aqueous hydrochloric acid (0.9 ml) and heated with stirring at 75°C for 114 hours to give orange-colored crystals. After the resulting solution was cooled down to room temperature, the crystals were filtered, washed three times with ethanol (1 ml) and dried *in vacuo* at 50°C for 72 hours to give an orange-colored crystal compound 9 (279 mg).
ESI (m/z): 624 (M-H)⁻
¹H-NMR(400 MHz, DMSO-d6, δppm):11.18(1H, s), 9.83(2H, s), 8.85(2H, d, J=6.2Hz), 8.84 (1H, d, J=8.4Hz), 8.76 (1H, d, J=8.4Hz), 8.27 (2H, d, J=6.2Hz), 7.17 (1H, d, J=1.1Hz), 6.97 (1H, d, J=1.9Hz), 6.83(1H, dd, J=8.4, 1.1Hz), 6.81 (1H, dd, J=8.4,1.9Hz), 5.96(1H, d, J=8.8Hz), 4.64 (2H, s), 3.75-4.03(4H, m), 3.42-3.55(2H, m)
IR (ATR): 3312, 1740, 1666, 1616, 1576, 1481, 1381, 1319, 1190, 1159, 1123, 1074, 739, 613 cm⁻¹
Purity: 98.2%

The above purity data were measured under the same condition as in Example 1-1.

**Table 8 Powder X-Ray Diffraction Data**

| 2θ | Diffraction intensity (cps) |
|---|---|
| 4.9 | 42 |
| 13.8 | 21 |
| 14.8 | 41 |
| 15.4 | 39 |
| 16.1 | 11 |
| 16.9 | 15 |
| 19.7 | 30 |
| 22.0 | 13 |
| 22.2 | 12 |
| 23.2 | 12 |
| 23.5 | 14 |
| 23.7 | 16 |
| 24.3 | 16 |
| 24.9 | 21 |
| 25.9 | 100 |
| 26.4 | 47 |
| 27.0 | 20 |
| 27.5 | 31 |
| 27.7 | 39 |
| 28.6 | 12 |
| 29.8 | 14 |
| 30.5 | 11 |
| 31.8 | 13 |
| 32.5 | 17 |
| 32.8 | 15 |
| 33.9 | 12 |
| 36.3 | 14 |
| 36.8 | 12 |
| 40.0 | 12 |
| 40.6 | 11 |

| | |
|---|---|
| Note: Each intensity is a relative value when the maximum value is 100. | |

The above powder X-ray diffraction analysis data were measured under the same condition as in Example 1-1.

### Quantitative determination of ion:

Hydrochloric acid 0.97 molar ratio

The above data for quantitative analysis of ion was measured under the same condition as in Example 2-1.

Residual solvent: no ethanol was detected.

The above data for residual solvent analysis was measured under the same condition as in Example 1-1.

Referential Example: An example of highly purifying the compound represented by the formula (II) by means of crystallization

The non-purified compound (II) (purity: 88%) prepared by a process shown in Example 14 of JP-A-10-245390 was crystallized by the Manufacturing Method 2 to give the compound (II) having a purity of 98%.

### Industrial Applicability

The compound represented by the formula (I) in a crystalline form shows significantly excellent stability, solubility, etc. as compared with the corresponding amorphous compound and, accordingly, it can be said that the compound is industrially applicable as an active ingredient for anti-cancer agent.

## Claims

1. Crystalline 6-N-pyridylmethylamino-12,13-dihydro-12-β-D-glucopyranosyl -5H-indolo[2,3-a]pyrrolo[3,4-c]carbazole-5,7(6H)-dione that is a free base having the following formula (I): 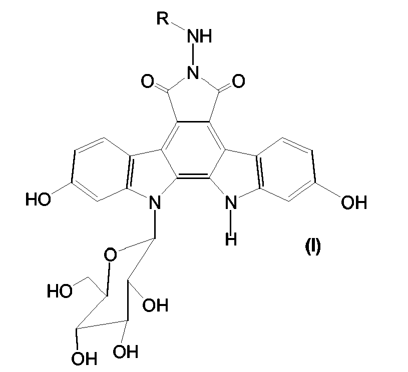 wherein R is an unsubstituted pyridyl methyl group or a pyridyl methyl group substituted by a hydroxy methyl group,
or a pharmaceutically acceptable salt thereof or a solvate thereof.

2. The crystalline free base according to Claim 1, a pharmaceutically acceptable salt thereof or a solvate thereof,
wherein the compound of formula (I) is represented by a compound of the following formula (II): 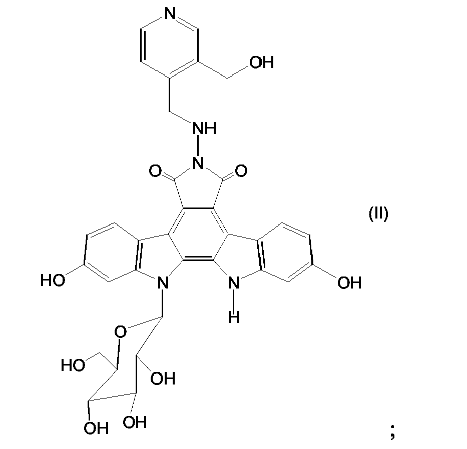 a compound of the following formula (III): 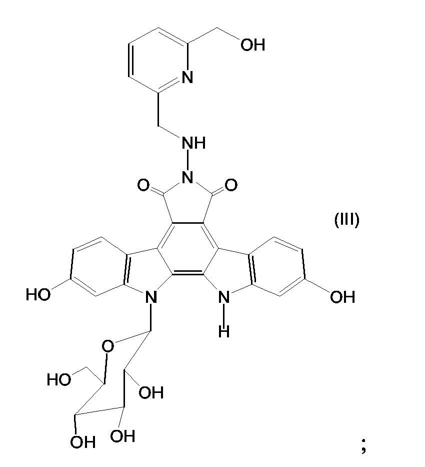 or a compound of the following formula (IV):

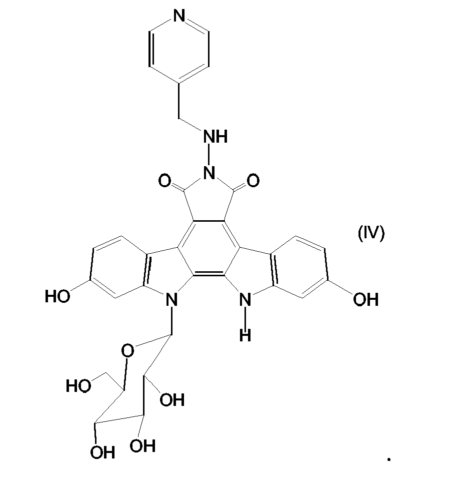

3. A pharmaceutically acceptable salt of the crystalline free base according to Claim 1, wherein:
the compound of formula (I) is represented by a compound of formula (II) as defined in Claim 2 and the pharmaceutically acceptable salt is hydrochloride, sulfate salt, or methanesulfonate salt;
the compound of formula (I) is represented by a compound of formula (III) as defined in Claim 2 and the pharmaceutically acceptable salt is hydrochloride, sulfate salt, or methanesulfonate salt; or
the compound of formula (I) is represented by a compound of formula (IV) as defined in Claim 2 and the pharmaceutically acceptable salt is hydrochloride, sulfate salt, or methanesulfonate salt.

4. The solvate according to Claim 1, wherein the compound of formula (I) is represented by a compound of formula (II), formula (III), or formula (IV) as defined in Claim 2.

5. The solvate according to Claim 1 which is ethanol solvate,
wherein the compound of formula (I) is represented by a compound of formula (II) as defined in Claim 2 and the pharmaceutically acceptable salt is methanesulfonate salt.

6. A process for manufacturing the crystalline compound of formula (I) according to Claim 1 which comprises:
the step of adding to an amorphous compound of formula (I) an organic solvent solution optionally containing one to one hundred equivalent of the corresponding acid or an appropriate acetic acid so that a concentration of the solution amounts to from 50 mg/l to 1g/l;
the step of refluxing the resulting solution with heating;
if desired, the step of filtrating the resulting solution to remove an insoluble matter;
the step of evaporating the solvent from the resulting solution to condense;
the step of refluxing a suspension of the resulting solid with heating;
the step of cooling the resulting suspension to a temprerature ranging from 0 degree to 35 degree; and the step of isolating the resulting crystal.

7. A process for manufacturing the crystalline compound of formula (I) according to Claim 1 which comprises:
the step of suspending an amorphous compound of formula (I) with an organic solvent solution optionally containing one to one hundred equivalent of the corresponding acid or an appropriate acetic acid;
the step of stirring the resulting solution with heating from 40 degree to 130 degree;
the step of cooling the resulting suspension to a temperature ranging from 0 degree to 35 degree; and
the step of isolating the resulting crystal.

8. A process for manufacturing the crystalline compound of formula (I) according to Claim 1 which comprises:
the step of adding to an amorphous compound of formula (I) water or a solution containing one to one hundred equivalent of the corresponding acid;
the step of stirring the resulting solution with heating from 40 degree to 130 degree;
if desired, the step of filtrating the resulting solution to remove an insoluble matter;
the step of cooling the resulting solution to a temperature ranging 0 degree to 35 degree with stirring; and the step of isolating the resulting crystal.

9. A pharmaceutical composition comprising as an active ingredient the crystalline free base of formula (I) according to Claim 1, a pharmaceutically acceptable salt thereof, or a solvate thereof, together with a pharmaceutically acceptable carrier or diluent.

10. An anti-tumor agent comprising as an active ingredient the crystalline free base of formula (I) according to Claim 1, a pharmaceutically acceptable salt thereof, or a solvate thereof, together with a pharmaceutically acceptable carrier or diluent.

11. An anti-tumor agent for injection using the crystalline free base of formula (I) according to Claim 1, a pharmaceutically acceptable salt thereof, or a solvate thereof

12. The anti-tumor agent for injection according to Claim 11, wherein the compound of formula (I) is represented by the compound of formula (II), formula (III), or formula (IV) of Claim 2.
